# EUROPEAN PATENT APPLICATION

(11) **EP 3 970 728 A1**
(43) Date of publication of application: **23.03.2022**
(21) Application number: 20805604.4
(22) Date of filing: 16.04.2020
(51) Int. Cl.: A61K 31/7048, A61K 45/06, A61P 29/00, A61P 11/00

(54) **DRUG AND COMBINATION PRODUCT USED FOR PREVENTION, ALLEVIATION AND/OR TREATMENT OF FIBROSIS, AND USE THEREOF**

(30) Priority: 16.05.2019 CN 201910409332
(71) Applicant: Shenyang Fuyang Pharmaceutical Technology Co., Ltd., Shenyang, Liaoning 110164 (CN)
(72) Inventor: HE, Hongwei, Shenyang, Liaoning 110164 (CN); JIANG, Enhong, Shenyang, Liaoning 110164 (CN); HE, Weiqing, Shenyang, Liaoning 110164 (CN); JIANG, Xundong, Shenyang, Liaoning 110164 (CN)
(74) Representative: Murgitroyd & Company
(86) International application number: PCT/CN2020/085111
(87) International publication number: WO 2020/228477

(57) **Abstract**

A medicament for preventing, alleviating and/or treating fibrosis comprises one of Carrimycin, Isovalerylspiramycin I, Isovalerylspiramycin II and Isovalerylspiramycin III; or a combination of two or three of Isovalerylspiramycin I, Isovalerylspiramycin II and Isovalerylspiramycin III. A combination product for preventing, alleviating and/or treating fibrosis comprises a first drug, an effective component of the first drug comprises one of Carrimycin, Isovalerylspiramycin I, Isovalerylspiramycin II and Isovalerylspiramycin III; or a combination of two or three of Isovalerylspiramycin I, Isovalerylspiramycin II and Isovalerylspiramycin III. It also relates to use of the medicine and combination product in preventing, alleviating and/or treating fibrosis.

## Description

### TECHNIAL FIELD

The present disclosure belongs to the field of medicinal chemistry, and in particular relates to medicament and combination product used for preventing, alleviating and/or treating fibrosis, and use thereof.

### BACKGROUND

Fibrosis is a pathological change, manifested by fibroblast activation and proliferation, increased fibrous connective tissue in tissues and organs, and decreased parenchymal cells. The continued progress of fibrosis can cause structural damage and loss of function of tissues and organs. Fibrosis of important organs seriously affects the quality of life of patients, and even life-threatening. Worldwide, tissue fibrosis is the main cause of disability and death caused by many diseases. According to relevant statistics in the United States, about 45% of the patients who died from various diseases in this country can be attributed to disease of tissue fibroplasia. At present, the treatment methods and drugs for this disease are still very lacking, and the prognosis is very poor. The development of new drugs that can effectively treat fibrosis is a very important and urgent task.

Carrimycin, also known as Bitespiramycin and Shengjimycin, is a new type of antibiotic with 4"-isovalerylspiramycin as a main component, formed by cloning the 4"-isovaleryl transferase gene (4"-o-acyl-transferase) of carbomycin-producing bacteria into spiramycin producing bacteria through transgenic technology, directionally acylating spiramycin 4"-OH, and adding an isovaleryl side chain at the 4" position under the collaboration between the Institute of Biotechnology of the Chinese Academy of Medical Sciences and the applicant.

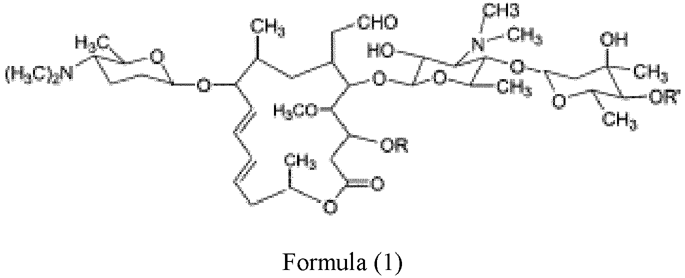

When R=H, R*'*=COCH₂CH(CH₃)₂, the compound is Isovalerylspiramycin I;
When R=COCH₃, R*'*-COCH₂CH(CH₃)₂, the compound is IsovalerylspiramycinII;
When R=COCH₂CH₃ , R*'*=COCH₂CH(CH₃)₂, the compound is Isovalerylspiramycin III.

The total content of the main active ingredient of isovalerylspiramycins (I+II+III) in Carrimycin is not less than 60%, and the total content of acylated spiramycin not less than 80%, and it is an acceptable pharmaceutical composition in pharmacy. The central structure of the Carrimycin is a 16-membered lactone ring, which is connected with a molecule of forosamine, a molecule of mycaminose, and a molecule of mycarose. Its main components, isovalerylspiramycins I, II, III, structurally differ from spiramycin in that the group attached to the 4" position of mycarose is isovaleryl instead of hydroxyl. The chemical structure of the Carrimycin, as shown in a formula (1), contains more than ten kinds of components. At present, the composition standard of the finished product of Carrimycin is that Isovalerylspiramycin III is ≥ 30%, the total ratio of Isovalerylspiramycin I, II, III is ≥ 60%, the proportion of total acylated spiramycin is ≥ 80%, and the sum of other unknown components is ≤ 5%.

Carrimycin is a 16-membered macrolide antibiotic with active groups of carboxyl, alkoxy, epoxy, ketone and aldehyde groups and a pair of conjugated C=C, with molecular weight of about 884-982. Due to the similar chemical structure, Carrimycin and macrolide antibiotics have a lot in common: they are easily soluble in most organic solvents such as esters, acetone, chloroform, and alcohols, and are slightly soluble in petroleum ether, and insoluble in water; their molecular structures contain two dimethylamine groups and is weakly alkaline, and thus they are easily soluble in acidic aqueous solutions; they have a "negative solubility" quality that decreases in solubility with the increasing temperature. Because the main component of Carrimycin, isovalerylspiramycin, has a longer carbon chain at the 4" position, it has a poor hydrophilicity, and its solubility in water is less than that of spiramycin and 4"-acetylspiramycin.

This drug has good lipophilicity, strong tissue penetration ability, fast oral absorption, long-term maintenance in the body, and sustained post-antibiotic effect. According to the relationship between the efficacy and the chemical conformation, after the acylation of the macrolide antibiotic at the 4" position, its lipophilicity and in vivo activity are improved, the in vivo antibacterial activity and clinical treatment effect have been significantly improved, and the stability of the antibiotic in the body is also enhanced with the growth of the carbon chain of the 4"-hydroxy ester, i.e., isovalerylspiramycin> butyrylspiramycin> propionylspiramycin> acetylspiramycin.

Preliminary in vivo and in vitro pharmacodynamic tests show that the drug not only has good antibacterial activity on most G+ bacteria, but also has a certain effect on some G- bacteria, and various technical indexes are obviously superior to azithromycin, erythromycin, acetylspiramycin and midecamycin, especially has the strongest antibacterial activity on mycoplasma pneumoniae, and has certain antibacterial activity on erythromycin resistant bacteria, neisseria gonorrhoeae, pneumococcus, staphylococcus aureus, pesudomonas pyocyaneum, himophilus influenzae, haemophilus influenzae, bacteroides fragilis, legionella, bacillus multiforme and clostridium perfringens, and has little cross resistance to erythromycin resistant staphylococcus aureus clinically. Carrimycin will be mainly used to treat Gram-positive bacteria infectious diseases, especially upper respiratory infection, and may be used for urinary system infection, etc.

Pharmacokinetic research results show that the active effective components in Carrimycin are mainly isovalerylspiramycins I, II and III. Carrimycin is rapidly metabolized into spiramycin after entering the body, and its oral absolute bioavailability is 91.6% on average based on the AUC₀₋ₜ sum of the parent drugs isovalerylspiramycins I, II, III and the active metabolites spiramycins I, II and III. The elimination of Carrimycin is slower after a single dose, and T_{1/2β} is between 23 and 27 hours.

From the end of 2019 to January 2020, pneumonia of unknown cause occurred in some areas, and the bronchoalveolar lavage specimens of the patients were submitted for next-generation sequencing, and a new type of coronavirus was found. On February 12, 2020, the WHO named the disease caused by the novel coronavirus (SARS-CoV-2): Corona Virus Disease 2019, COVID-19. For lung damage caused by novel coronavirus infection, including pulmonary fibrosis, it is urgent to find effective treatment drugs and drugs to improve lung symptoms.

So far, there has been no record or report on the treatment of fibrosis with Carrimycin.

In view of that, the present disclosure has been proposed.

### SUMMARY

The technical problem to be solved by the disclosure is to overcome the defects of the prior art and the disclosure provides a medicament for preventing, alleviating and/or treating fibrosis. The medicament can effectively prevent, relieve and treat fibrosis, and has significant social and economic benefits.

To solve the above technical problems, the basic idea of the technical solution adopted by the present disclosure is as follows:
The present disclosure provides a medicament for preventing, alleviating and/or treating fibrosis, and an effective component of the medicament comprises one of Carrimycin, Isovalerylspiramycin I, Isovalerylspiramycin II and Isovalerylspiramycin III;
or a combination of two or three of Isovalerylspiramycin I, Isovalerylspiramycin II and Isovalerylspiramycin III.

Furthermore, the medicament comprises a pharmaceutically acceptable carrier.

Furthermore, the medicament is prepared into pharmaceutically acceptable tablets, capsules, pills, injections, sustained-release agents and various microparticle delivery systems.

The present disclosure provides a medicament for preventing, alleviating and/or treating fibrosis, an effective component of the medicament comprises one or more selected from a group consisting of:
a derivative, a pharmaceutically acceptable salt, a solvate, a metabolite, a stereoisomer, a tautomer, a polymorph and a drug precursor of Carrimycin;
a derivative, a pharmaceutically acceptable salt, a solvate, a metabolite, a stereoisomer, a tautomer, a polymorph and a drug precursor of Isovalerylspiramycin III;
a derivative, a pharmaceutically acceptable salt, a solvate, a metabolite, a stereoisomer, a tautomer, a polymorph and a drug precursor of Isovalerylspiramycin II; and
a derivative, a pharmaceutically acceptable salt, a solvate, a metabolite, stereoisomer, a tautomer, a polymorph and a drug precursor of Isovalerylspiramycin I.

The present disclosure provides a combination product for treating fibrosis comprising a first drug, and an effective component of the first drug comprises one of Carrimycin, Isovalerylspiramycin I, Isovalerylspiramycin II and Isovalerylspiramycin III;
or a combination of two or three of Isovalerylspiramycin I, Isovalerylspiramycin II and Isovalerylspiramycin III.

Furthermore, the combination product further comprises a second drug, and the second drug comprises at least one of medicaments for preventing, alleviating and/or treating fibrosis.

Furthermore, the medicaments for preventing, alleviating and/or treating fibrosis include corticosteroids, colchicine, silymarin, or interferon.

The present disclosure provides a combination product for treating fibrosis comprising a first drug, an effective component of the first drug comprises one or more selected from a group consisting of:
a derivative, a pharmaceutically acceptable salt, a solvate, a metabolite, a stereoisomer, a tautomer, a polymorph and a drug precursor of Carrimycin;
a derivative, a pharmaceutically acceptable salt, a solvate, a metabolite, a stereoisomer, a tautomer, a polymorph and a drug precursor of Isovalerylspiramycin III;
a derivative, a pharmaceutically acceptable salt, a solvate, a metabolite, a stereoisomer, a tautomer, a polymorph and a drug precursor of Isovalerylspiramycin II; and
a derivative, a pharmaceutically acceptable salt, a solvate, a metabolite, stereoisomer, a tautomer, a polymorph and a drug precursor of Isovalerylspiramycin I.

The fibrosis in the invention includes pulmonary fibrosis, cardiac fibrosis, liver fibrosis, pancreatic fibrosis, kidney fibrosis, bone marrow fibrosis and skin fibrosis.

In one embodiment, the pulmonary fibrosis includes pulmonary fibrosis caused by a novel coronavirus infection. The medicines and combination products of the present application have ameliorating effects on pulmonary fibrosis caused by the novel coronavirus (SARS-CoV-2) infection.

When lung damage is caused by multiple reasons, the interstitium secretes collagen for repair. If it is repaired excessively, the excessive proliferation of fibroblasts and the accumulation of extracellular matrix form pulmonary fibrosis.

Myocardial fibrosis is formed by excessive proliferation of cardiac interstitial fibroblasts, excessive deposition and abnormal distribution of collagen.

Liver fibrosis is a pathological process in which various pathogenic factors cause abnormal proliferation of connective tissue in the liver and excessive precipitation of diffuse extracellular matrix in the liver. Many factors can cause liver fibrosis, such as viral infection, inflammation, oxidative stress, and alcoholism.

Regarding the pancreatic fibrosis, a large amount of protein secreted by pancreatic acinar cells, while the fluid and bicarbonate secreted by pancreatic duct cells are not increased, resulting in a decrease in the concentration of Lithostathine and GP2(A protein that can form a cast) secreted by pancreatic acinar cells, and precipitation in the pancreatic duct, causing pancreatic fibrosis.

Kidney fibrosis is a pathological process in which extracellular matrix and inappropriate connective tissue accumulate in the kidney, leading to changes in kidney structure and impaired function.

Bone marrow fibrosis is a myeloproliferative disease caused by the proliferation of collagen in the hematopoietic tissue of the bone marrow, and the hematopoietic function seriously affected by the fibrous tissue.

Skin fibrosis is formed when fibroblasts divide, proliferate, migrate to the damaged part, produce extracellular matrix, and form scar tissue to repair the wound under conditions such as trauma. Scar formation is a process of gradual fibrosis of granulation tissue.

The present disclosure further provides a use of the medicament or the combination product in preventing, alleviating and/or treating fibrosis.

The present disclosure further provides a use of the medicament or the combination product in inhibiting inflammation or lipid peroxidation, inhibiting the proliferation and activation of fibroblasts, and promoting collagen degradation.

After adopting the above technical solution, compared with the prior art, the present disclosure has the following beneficial effects:
The medicines and combination products provided by the invention have good therapeutic effects in treating fibrosis, and have important social and economic benefits.

The specific embodiments of the present disclosure will be described in further detail with reference to the accompanying drawings.

### BRIEF DESCRIPTION OF THE DRAWINGS

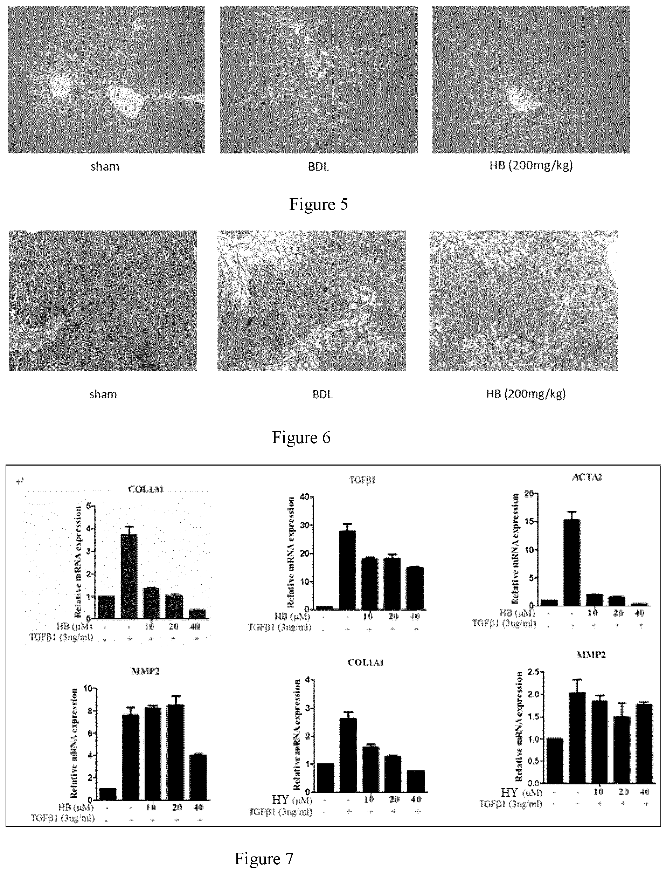

Fig. 1 shows the inhibitory effects of HB (Carrimycin) and HY (Isovalerylspiramycin I) on the activity of type I collagen α1 promoter;
Fig. 2 shows the IC50 values of HB (Carrimycin) and HY (Isovalerylspiramycin I) on HepG2 and LX2 cells;
Fig. 3 is the results of Real-time PCR, showing the effects of HB (Carrimycin) and HY (Isovalerylspiramycin I) on the mRNA levels of major fibrosis markers in LX-2 cells induced by TGFβ1;
Fig. 4 is the results of Western Blot detection, showing the effects of HB (Carrimycin) and HY (Isovalerylspiramycin I) on the protein levels of major fibrosis markers in LX-2 cells induced by TGFβ1;
Fig. 5 shows the effect of HB (Carrimycin) on the pathological structure of rat liver tissue;
   Among them, in Figure 5: sham is the H&E stained section of the liver tissue of the sham-operated group; BDL is the H&E stained section of the liver tissue of the BDL model group; HB is the H&E stained section of the liver tissue of the Carrimycin administration group result;
Fig. 6 shows the effect of HB (Carrimycin) on the degree of fibrosis in rats;
   Among them, in Figure 6: sham is the result of Masson stained section of liver tissue of rats in the sham operation group; BDL is the result of Masson stained section of liver tissue of rats in the BDL model group; HB is the result of Masson stained section of liver tissue of rats in Carrimycin administration group;
Fig. 7 is the results of Real-time PCR, showing the effects of Carrimycin and Isovalerylspiramycin I on the mRNA levels of the main fibrosis markers in lung fibroblasts MRC-5 induced by TGFβ1; among them, HB is Carrimycin, HY is Isovalerylspiramycin I;

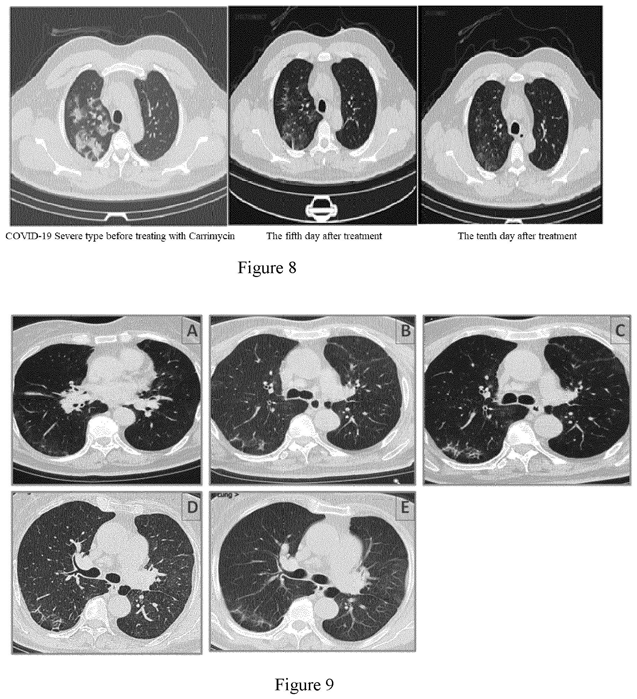

Fig. 8 shows the improvement of the lungs before and after treatment with Carrimycin in patient 1;
Fig. 9 shows the improvement of the lungs before and after treatment with Carrimycin in patient 2;

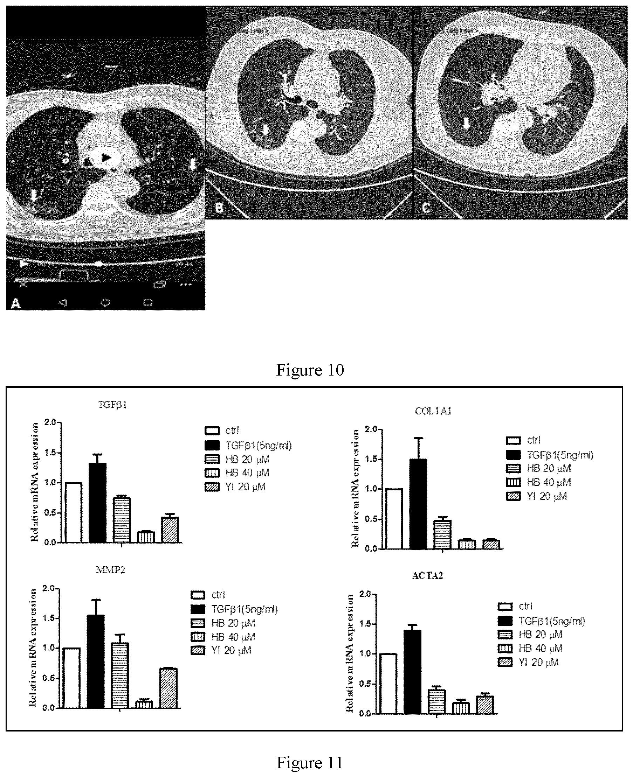

Fig. 10 shows the improvement of the lungs before and after treatment with Carrimycin in patient 3;
Fig. 11 is the results of Real-time PCR, showing the effect of Carrimycin (HB) and Isovalerylspiramycin I (YI) on the mRNA level of the main fibrosis markers in CCC-ESF-1 induced by TGFβ1 Influence.

It should be noted that these drawings and written descriptions are not intended to limit the conceptual scope of the present disclosure in any way, but to explain the concept of the present disclosure to those skilled in the art by referring to specific embodiments.

### Specific Embodiments

In order to make the objectives, technical solutions and advantages of the embodiments of the present disclosure clearer, the technical solutions in the embodiments will be described clearly and completely with reference to the drawings in the embodiments of the present disclosure. The following embodiments are used to illustrate the present disclosure, but are not intended to limit the scope of the present disclosure.

### Example 1: Medicament C tablets

Specification: 200mg/350mg

Prescription of the tablet core:

| | |
|---|---|
| Medicament C | 200g |
| microcrystalline cellulose | 110g |
| sodium starch glycolate | 22g |
| povidone K₃₀ (5%) | 15g |
| magnesium stearate | 3g |
| formulated into 1000 tablets | |

Prescription of the coating solution:

| | |
|---|---|
| Opadry II | 21g |
| Distilled water | proper amount |
| formulated into 105mL | |

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

### The preparation process:

Preparation of the tablet core: the main drug and the excipients respectively passed through a 100-mesh sieve, and a prescription dosages of raw powder of medicament C and microcrystalline cellulose with a 1/2 prescription dosage of sodium starch glycolate were uniformly mixed, then an aqueous solution of 5% povidone K₃₀ was added to prepare a soft material. An 18-mesh sieve was used for granulating, and the wet granules were dried under a ventilated condition at 60DEG C for 2h. After the wet granules were dried, a 18-mesh screen was used for dispersing the granules, then a 1/2 prescription dosage of sodium starch glycolate and magnesium stearate were added. And after the materials were uniformly mixed, the mixture was tableted by using a shallow concave die of a diameter of 11mm, to obtain a drug-containing tablet core with the tablet weight of 350mg and the hardness of 6.5kg.

Preparation of the coating solution: the required amount of Opadry II (white color) was weighed, the required amount of water was added into the preparation container in batches, after all of the water has been added, the stirring speed was reduced to make the spiral disappear, and the stirring was continued to be performed for 30min to obtain the coating solution.

Preparation of the film coated tablet: the tablet core was placed into a coating pan, the coating conditions were determined, and coating was carried out with the main rotation speed of 20r/min, the air inlet temperature of 40DEG C, the air outlet temperature of 30 DEG C, the atomization pressure of 0.02Mpa and the spraying flow rate of 1ml/min. And after a constant state was achieved, the coating was continuously to be sprayed for 1.5h to obtain a tablet with a smooth surface and a uniform colour and lustre. The tablet were qualified if it were in compliance with the inspecting standards of thin-film coating. The coating adds the weight by approximately 5%.

### Example 2: medicament C plain tablets (calculated for 10000 tablets)

Prescription:

| | |
|---|---|
| medicament C | 1000g |
| low-substituted hydroxypropyl cellulose (5%) | 92.5g |
| sodium starch glycolate (3%) | 55.5g |
| magnesium stearate (1%) | 18.5g |
| the total weight of starch subtracts the weights of the other raw materials and excipients | 850g |

formulated into 10000 tablets

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

The preparation process: a proper amount of starch was weighed, diluted to a concentration of 15%, and heated to be a paste, to obtain an adhesive. the main material, row powder of medicament C, and the excipients starch, low-substituted hydroxypropyl cellulose, sodium starch glycolate and magnesium stearate passed through a 100-mesh sieve, respectively; and prescription dosages of the main material and the excipients were weighed. After the raw powder of medicament C, starch and low-substituted hydroxypropyl cellulose were fully and uniformly mixed, the starch paste with the starch concentration of 15% was used to prepare the mixture into a soft material which was granulated by a 14-mesh sieve, and granules were dried at 50-60 DEG C to control the moisture content at 3-5%. A 14-mesh sieve was used for dispersing the granules, and then sodium carboxymethyl starch and magnesium stearate were added to be mixed, and the granule content was measured. The weight of the tablet was calculated according to the granule content, and the mixture was tableted (with a Φ9 mm shallow concave punch), then the difference in the weight of the tablets was detected. After passing the test, the tablets were packaged.

### Example 3: medicament C capsules (calculated for 10000 granules)

Prescription:

| | |
|---|---|
| medicament C | 1000g |
| starch | 1080 subtracts the weight of medicament C |
| medicinal No. 3 capsule | 1000 granules |
| liquid paraffin | 50ml |
| formulated into 10000 granules | |

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

The preparation process: the main material, raw powder of medicament C, and the excipient medicinal starch were separately weighed according to the dosages of the process prescription, and then fully mixed infor 1.5-2 hours. The data obtained by sampling and detecting the content should be substantially consistent with the theoretical data (the weight contained by each of the capsules was approximately 0.105g); and the qualified No. 3 medicinal capsule and the mixed raw materials to be loaded were filled in a filling device according to the operation requirements of an automatic capsule machine, and the filled capsules were subjected to a difference test (±10% or less, <0.3g) to see if the dissolution rate meets the requirements or not. The capsules that meet the requirements after being tested were put into a polishing machine to be polished for 15-20 minutes with the liquid paraffin added, and then were taken out to be tested by finished product packaging boxes.

### Example 4: medicament C dried syrup (calculated for 10000 bags)

Prescription:

| | |
|---|---|
| medicament C | 1250g |
| citric acid (0.5%) | 15g |
| sucrose | the total weight subtracts the weights of the |
| other raw materials and excipients | |
| total weight, approximately | 500g |
| pigment (Curcumin) | approximately 1g |
| formulated into 10000 bags | |

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

The preparation process: the raw powder of medicament C, citric acid and sucrose were respectively grinded into granules by using a jet-stream pulverizer, and 85% of the granules passed through a 300-mesh sieve, 15% of the granules passed through a 180-mesh sieve. Then the fine powder after grinding was weighed according to the prescription amount and fully mixed for 1-1.5 hours, the content was measured, the loading capacity was calculated (the theoretical filling amount was 500mg per bag). Then the mixture was put into a bagging machine, aluminum foil paper was installed, and filling was carried out according to the operation requirements of a filling machine. The difference was allowed to be within ±5 %, and after the filling, the outer packaging was carried out after passing the inspection.

### Example 5: medicament C granule preparation (calculated for 10000 bags)

Prescription:

| | |
|---|---|
| medicament C | 1250g |
| sugar powder | 20000g |
| dextrin | 9000g |
| 5% PVP-K₃₀ | proper amount |
| formulated into 10000 bags | |

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

The preparation process: the raw powder of medicament C, sugar powder and dextrin passed through a 120-mesh sieve, and the raw powder of medicament C, sugar powder and dextrin were weighed according to the prescription amount and uniformly mixed. And the above uniformly mixed materials were made into a soft material with a 5% PVP-K₃₀ mucilage, and then the soft material was granulated with a swinging granulation machine, dried at 70 DEG C and subjected to granule dispersion, and the resulting granules were subpackaged after being qualified for inspection.

### Example 6: freeze-dried powder injection

The process: 500mg of raw powder of medicament C was uniformly mixed with an equal molar amount of hexanedioic acid, and the mixture was dissolved in 5 ml of water to obtain a faint yellow clear solution having a pH between 4.6 and 5.6. Further, 40 mg of mannitol was added as a lyophilized proppant into the faint yellow clear solution, and after being frozen rapidly at a low temperature for 9 hours, the material was freeze-dried to obtain a faint yellow loose mass, which was dissolved in 10 ml of sterile water before being used.

The medicament C is one or more of Carrimycin, Isovalerylspiramycin III, Isovalerylspiramycin II or Isovalerylspiramycin I, or is one or more of corresponding derivatives, pharmaceutically acceptable salts, solvates, metabolites, stereoisomers, tautomers, polymorphs, or drug precursors.

### Experimental example 1 The effect of medicament C in anti-liver fibrosis

The present invention uses human hepatic stellate cell line LX-2 as the research object in vitro, and uses Real-time PCR and Western Blot as research methods to confirm that medicament C has an inhibitory effect on the mRNA and protein levels of the main fibrosis maker in LX-2 cells induced by TGFβ1. At the same time, in order to further determine the anti-fibrosis effect of medicament C, the present invention uses the common bile duct ligation rat fibrosis model, and detects the pathological changes of rat liver tissue after oral administration of medicament C. The results show that medicament C can effectively relieve Pathological changes and degree of fibrosis in the rat liver caused by bile duct ligation.

### 1. Medicament C (using Carrimycin or Isovalerylspiramycin I) inhibits the activity of COL1A1 promoter-luciferase reporter gene.

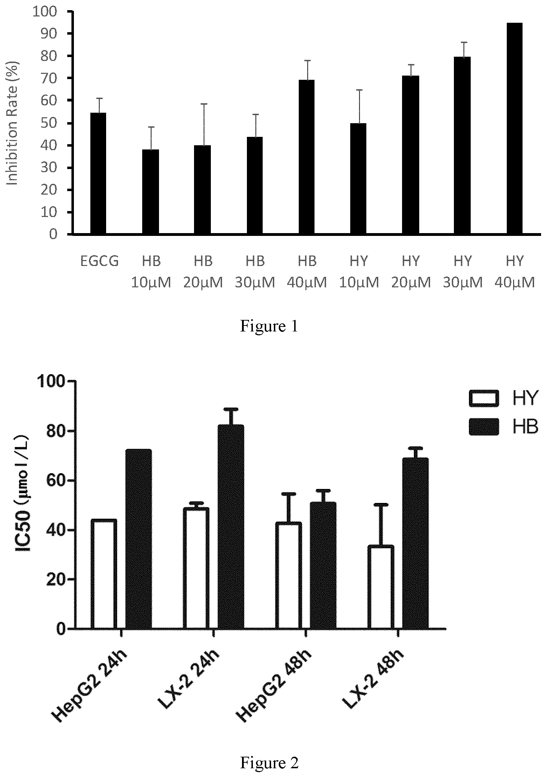

The constructed monoclonal cells LX2-COL stably expressing the type I collagen α1 promoter COL1A1P were spread on a 96-well plate with 2×10⁴ cells per well. After the cell confluence is about 90%, different concentrations of medicament C (using Carrimycin or Isovalerylspiramycin I) were added, and each group of experiments has 4 replicate holes. Follow the instructions of Bright-Glo^{™} Luciferase Assay System, the medium in wells were removed after 24 hours, and 50 µl/well of any medium was added to each well. 50 µl of luciferase substrate was added to each well, and detection was performed after 2 min. The results showed that as the concentration of medicament C was increased, the fluorescence intensity was decreased. When the concentrations of Carrimycin or Isovalerylspiramycin I was 40 µM, the fluorescence intensity was decreased most significantly (Figure 1). Figure 1 showed the inhibitory effect of Carrimycin (HB) and Isovalerylspiramycin I (HY) on the activity of type I collagen α1 promoter. This result showed that Carrimycin and its single-component Isovalerylspiramycin I have a significant inhibitory effect on the activity of the COL1A1 promoter.

### 2. Medicament C (using Carrimycin or Isovalerylspiramycin I) inhibits the proliferation of human hepatocytes HepG2 and human hepatic stellate cells LX-2.

Human liver cancer HepG2 cells and human hepatic stellate cells LX-2 were plated on 96-well plates and cultured, with 4×10³ cells per well. After 24 hours, different concentrations of medicament C were added (using Carrimycin (HB) or Isovalerylspiramycin I (HY)), and each group of experiments had 3 replicate holes. After the cells were treated with drugs for 24 or 48 hours, they were stained by the sulforhodamine (SRB) method, and the absorbance at 515 nm was measured with a microplate reader to calculate the half inhibition rate (IC50). The results showed that Carrimycin and its single-component Isovalerylspiramycin I had a certain inhibitory effect on the proliferation of the HepG2 and LX-2 cells, with IC50 between 10µM and 100µM (Figure 2).

### 3. Medicament C (using Carrimycin or Isovalerylspiramycin I) inhibits the expression of the main markers of fibrosis in LX-2 cells induced by TGFβ1 at the mRNA and protein levels

TGFβ1 induced LX-2 cells and medicament C (using Climycin (HB) or Isovalerylspiramycin I (HY)) administration treatment: LX-2 cells were cultured in DMEM, High Glucose, GlutaMAX^{™} (Gibco10566016) medium containing 10% fetal bovine serum and mixture of 1% Streptomyces and 1% penicillin at 37 DEG C and 5% CO₂. 1×10⁵ cells per well were plated on a 6-well plate. After 24 hours of culture, the original medium in the 6-well plate was removed by a vacuum pump and added with DMEM medium without 10% fetal bovine serum. After starvation for 24 hours, TGF- β1 (2 ng/ml) was added to induce cell, and different concentration gradients of Carrimycin or Isovalerylspiramycin I were added at the same time, the concentrations were 10 µmol/L and 20 µmol/L respectively. A control group (without adding TGF- β1 induction), TGF-β1 induction group (only TGF-β1 induction) and TGF-β1 induction administration group (both TGF-β1 induction and medicament C treatment) were set.

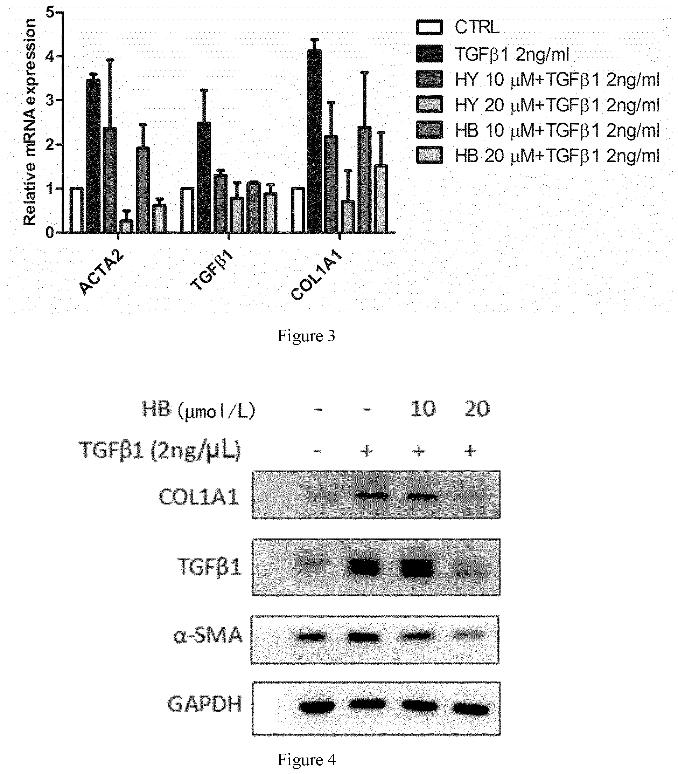

After culturing for 24 hours, the medium was discarded, and the total RNA of LX-2 cells were extracted according to the TRIzol instructions. Following the instructions of Roche Transcriptor First Strand cDNA Synthesis Kit, the LX-2 total RNA were to reverse transcribed into cDNA. The obtained cDNA, sterile water, Roche FastStart Universal Probe Master (Rox) and ABI TaqMan probes (GAPDH, COL1A1, TGFB1, ACTA2) were prepared into a 20 µl reaction system, and the ABI 7500 Fast Real-Time PCR System was used for detection. GAPDH was used as an internal control to analyze the results. The data showed that with the increase of the doses of Carrimycin or Isovalerylspiramycin I, the expression of COL1A1, TGFB1, and ACTA2 decreased. And Carrimycin or Isovalerylspiramycin I at 20 µmol/L had the best inhibitory effect on COL1A1, TGFB1, and ACTA2 at the mRNA level (Figure 3).

After the cells were induced by TGFβ1 and continued to be cultured for 24 hours, 1 ml of Ripa lysis solution (containing 1% PMSF) was added to each well of the 6-well plate to extract the protein. And the protein concentration was determined by the BCA method. The sample was loaded at 25 µg/20 µl per well. After electrophoresis, transfer membrane, 5% skim milk blocking, and antibody incubation, the desired protein bands was observed by using the Tianneng 5200 imaging system. The results showed that the expression of COL1A1, TGFβ1, and α-smooth muscle actin decreased with the increase of the concentration of Carrimycin. And the expression of the above-mentioned marker proteins could be significantly inhibited when the Carrimycin or Isovalerylspiramycin I was at 20 µmol/L(Figure 4).

### 4. Preparation of SD rat common bile duct ligation induced fibrosis model

Twelve male SD rats weighing 180-220 g were selected and randomly divided into sham operation group, model group, and medicament C administration group, with 4 rats in each group. Before the animal experiment and after fasting and water for 12 hours, the animals were anesthetized with isoflurane for surgery. The model group and the drug administration group underwent common bile duct ligation (BDL). In a sterile operating table, a midline incision was made in the upper abdomen, the liver margin was raised, the duodenum was opened, and the common bile duct was separated by 2-3 cm. Two lines with No. 000 surgical sutures were used to ligate at the place near the duodenum and near the hilum respectively, and the common bile duct was cut off from the middle. In the sham operation group, only the midline incision of the upper abdomen was made and sutured, and the common bile duct was not ligated. After the animals were awake from anesthesia, they ate and drank normally and drank freely. Gavage was started on the second day after surgery, and normal saline (sham operation group, BDL model group) and Carrimycin or Isovalerylspiramycin I 200 mg/kg (medicament C administration group) were given, respectively, once a day for 14 consecutive days.

### 5. The improvement effect of medicament C (using Carrimycin) on the pathological structural changes of rat liver induced by BDL

Before sampling, the rats were fasted and water for 12 hours, and the rats were sacrificed. The liver tissues were taken, and the large hepatic lobe tissues were cut out and fixed in 10% formalin. After dehydration, paraffin embedding, slicing, baking slices, etc., paraffin sections are made. Hematoxylin-Eosin (H&E) staining solution was used for staining, and the changes in the pathological structure of rat liver tissue were observed under a microscope. The results showed that in the sham operation group, the liver cells of the liver tissue of the rats in the sham operation group were arranged neatly, the liver lobule structure was complete, and there was no bile duct hyperplasia. The pathological structure of the liver tissue of the rats after BDL changed significantly, the bile duct hyperplasia was very obvious, and the tissue necrosis was significantly increased. The pathological changes of the liver tissue structure of the rats in the Carrimycin administration group were relieved, the bile duct proliferation was inhibited, and the degree of tissue necrosis was significantly reduced (Figure 5). It showed that Carrimycin can significantly improve the pathological changes of liver tissue in BDL rats.

### 6. Inhibitory effect of medicament C (using Carrimycin) on fibrosis induced by BDL in rats

The paraffin sections were stained with Masson dye to observe the changes in liver fibrosis in rats. The results showed that the degree of liver fibrosis in rats after BDL increased significantly, and collagen deposition was serious. The fibrosis and collagen deposition were significantly inhibited after the administration of Carrimycin (Figure 6). It showed that Carrimycin can significantly inhibit BDL-induced fibrosis in rats.

### Experimental example 2 Function of medicine C in inhibit pulmonary fibrosis

Firstly, medicine C (using Carrimycin or Isovalerylspiramycin I) inhibits the expression of the main markers of fibrosis in lung fibroblasts MRC-5 induced by TGFβ1 at the mRNA and protein levels.

Method for TGFβ1 inducing lung fibroblasts MRC-5 and administrating Carrimycin or Isovalerylspiramycin I comprises: MRC-5 was cultured in a MEM (Gibco 11095-080) culture medium containing 10% fetal bovine serum, a mixture of 1% penicillin and 1% streptomycin and 1% non-essential amino acids at 37 DEG C and 5% CO₂, and was plated in a 6-well according to 3×10⁵ cells per well. After culturing for 24 hours, the original culture medium in the 6-well plate was removed by a vacuum pump, and MEM culture medium without 10% fetal bovine serum was added to be starvation culture for 24 hours. And then TGF- β1 (3ng/ml) induction was added, and at the same time different concentration gradients of Carrimycin and Isovalerylspiramycin I were added. The concentrations were 10µmol/L, 20µmol/L, 40µmol/L. The following groups were set, control group (without adding TGF -β1 induction), TGF-β1 induction group (only TGF-β1 induction is added) and TGF-β1 induction administration group (both adding TGF-β1 induction and treating with Carrimycin or Isovalerylspiramycin I).

After culturing for 24 hours, the culture medium was discarded, and the Total RNA of MRC-5 cells were extracted according to the operation steps of the TRIzol instructions. According to the operation steps of the instructions of Roche Transcriptor First Strand cDNA Synthesis Kit, Total RNA of LX-2 was reversely transcribed into cDNA. The cDNA, sterile water, Roche FastStart Universal Probe Master (Rox) and ABI TaqMan probes (GAPDH, COL1A1, TGFB1, ACTA2, MMP2) were prepared into a 20µl reaction system, and the ABI 7500 Fast Real-time PCR System was used for detection. GAPDH was used as an internal control to analyze the results. It is showed by the data, with the increase of the dose of Carrimycin, the expressions of COL1A1, TGFB1, ACTA2, and MMP2 were decreased, and the effect of inhibiting COL1A1, TGFB1, and ACTA2 was the best at the mRNA level when the Carrimycin or Isovalerylspiramycin I was at 40 µmol/L. With the increase of the dose of Isovalerylspiramycin I, the expression of COL1A1 was decreases, and the effect of inhibiting COL1A1 at the mRNA level at 40 µmol/L was the best (Figure 7).

Secondly, the improvement effect of Carrimycin on pulmonary fibrosis of pneumonia (Corona Virus Disease 2019, COVID-19) caused by a novel Coronavirus (SARS-CoV-2 ) infection.

The subjects were 18-75 years old and met the diagnostic criteria for pneumonia caused by novel coronavirus infection (Fifth Edition). The patients meet any of the following: (1) having a fever again or clinical symptoms being worsen, (2) negative being turned to positive by throat swab nucleic acid test, (3) clinical symptoms being not improved or the result of nucleic acid test being still positive, (5) pneumonia or the progress of fibrosis being shown on chest CT. SOFA score: 1 point to 13 points

### 1. Treatment method

Mild type: orally administrating 0.4g Carrimycin tablets each time after a meal, once a day, for 7 consecutive days, and entering the follow-up observation period for 30 days after the treatment.

Typical type: orally administrating 0.4g Carrimycin tablets each time after a meal, once a day, for 10 consecutive days, and entering the follow-up observation period for 30 days after the treatment.

Severe and critical type: orally administrating 0.4g Carrimycin tablets each time after a meal, once a day, and administrating by nasogastric tube those for those who cannot be taken orally. After the treatment, the follow-up observation period was entered for 30 days.

### 2. Main efficacy indexes

(1) Time for keeping not having fever (days).
(2) Time for keeping being without pulmonary symptom (HRCT) (days).
(3) The negative rate of novel coronavirus by throat swabs test on the third day and seventh day after treatment (%).

### 3. The overall situation of the patients

There were 47 patients of virus "positive by nucleic acid retest" or treated patients, including 11 patients of mild type, 27 patients of typical type, 3 patients of severe type, and 6 patients of critical type. After initial treatment, there were 40 patients being still positive for viral nucleic acid and 7 patients being negative for nucleic acid.

### 4. Evaluation of main curative effect

(1) Among 40 patients with positive viral nucleic acid, 16 patients had conversed to nucleic acid negative on the third day, 13 patients had conversed to nucleic acid negative on seventh day, 1 patient had conversed to nucleic acid negative on fifteenth day, and the remaining 10 patients had just joined the group and the nucleic acid of them was not rechecked.
(2) There were 19 patients with lung inflammation at the time of enrollment (7 patients with nucleic acid negative and 12 patient with nucleic acid positive), and the marked improvement rate of lung inflammation was 73.7% (14/19) on the seventh day.
(3) At the time of enrollment, there were 5 patients with fever (3 patients with nucleic acid negative cases and 2 patients with nucleic acid positive). The normalization rate of the body temperature on the third day was 60% (3/5), and the normalization rate of the body temperature return rate on seventh day was 100% (5/5).

### 5. Improvement of lung symptoms in main patients

CT manifestations of pulmonary fibrosis include:
1. The distribution of lesions is peripheral and subpleural.
2. The CT manifestations of pulmonary fibrosis are significantly different in different periods. At the early stage patchy ground glass shadows in the middle and lower lungs is shown in HRCT, which indicates active lesions and reversible lesions. At this time, the changes in interstitial lesions are not obvious.
3. Progression is developed to pulmonary fibrosis, CT shows grid shadows, HRCT shows irregular thickening of the interlobular septum, and the small blood vessels in the lobules becomes obvious due to the thickening of the wall. In the late stage, CT shows wide honeycomb shadows, and the structure of the lobules is deformed. Because bronchiectasis is caused by pulmonary fibrosis, the wide honeycomb shadows are most obvious under the pleura of the middle and lower lungs.
4. Ground glass shadow: It is an important sign of pulmonary fibrosis. The ground glass shadow means that the lesion is in an active phase and needs to be actively treated. It can be an interstitial or substantial lesion.
5. Honeycomb shadow: It is a smaller cystic shadow, of which most are in several millimeters to ten millimeters, and a few can reach several centimeters. Honeycomb shadow has thick and clear fibrous walls, and appears generally in the periphery of the lungs and under the pleura. The normal structure of the part with obvious honeycomb shadow is distorted, the lobular structure is unrecognizable, and the pleura that is usually connected with the honeycomb shadow is slightly thickened, which is a manifestation of interstitial fibrosis in the late stage.

Patients with novel coronavirus-infected pneumonia have improved their pulmonary symptoms and fibrosis after treatment with Carrimycin. The specific is as follows:
Figure 8 is CT images of the lungs of patient 1 before and after treating with Carrimycin. It can be seen from the figure that the pulmonary symptoms were significantly improved after 5 or 10 days of treatment with Carrimycin.

Figure 9 shows the changes in CT images of the lungs of patient 2. (A) is the CT scanning on the first day of illness, (B) is the CT scanning on the 5th day of illness, and (C) is the CT scanning on the 6th day of illness Scanning image (the day of starting Carrimycin treatment), (D) is the CT scanning on the 8th day of illness; (E) is the CT scanning on the 11th day of illness. After continuing to administrate Carrimycin for treatment, the condition of the lungs improved.

It is shown in Figure 10 that the changes in the CT images of patient 3, specifically, the patient is female and 72 years old. After admission, she was treated with oxygen inhalation through a nasal cannula, and orally administrated 0.4 g of Carrimycin once a day. On the second day after admission, the patient's general condition was improved, coughing and dyspnea were significantly improved, and oxygen saturation was increased to 98%. Oxygen partial pressure was increased by 130mmHg through blood gas analysis. Twice throat swabs were performed on the 3rd and 6th days after the treatment of Carrimycin, and the nucleic acid tests were all negative. It shown in CT image (Figure 10) that, on the 6th day of the illness course (1 day before taking Carrimycin), the markings of the bilateral lungs were increased, irregular ground-glass lesions were seen in the lower field of the right lung, and patchy shadows were scattered on the left side (shown by arrow A) ). On the 9th day of the illness course (3 days after taking Carrimycin), the markings of the bilateral lungs were clear, and the irregular ground-glass lesions in the lower field of the right lung were obviously absorbed (shown by B arrow). On the 12th day of the illness course (5 days after taking Carrimycin), a small amount of fibrosis was formed in the right lung lesion (shown by C arrow). After continuing to administrate Carrimycin for treatment, the fibrosis condition was improved.

### Experimental example 3 Function of medicine C in inhibit skin fibrosis

1. Medicine C (using Carrimycin or Isovalerylspiramycin I) inhibits the expression of the main markers of fibrosis in skin fibroblasts CCC-ESF-1 induced by TGFβ1 at the mRNA level.

Method for TGFβ1 inducing skin fibroblasts CCC-ESF-1 and administrating Carrimycin and Isovalerylspiramycin I comprises: CCC-ESF-1 was cultured in DMEM, High Glucose, GlutaMAX^{™} (Gibco 10566016) culture medium containing 10% fetal bovine serum, mixture of 1% penicillin and 1% streptomycin at 37 DEG C and 5% CO₂, and was plated in a 6-well according to 3×10⁵ cells per well. After culturing for 24 hours, the original culture medium in the 6-well plate was removed by a vacuum pump, and DMEM culture medium without 10% fetal bovine serum was added to be starvation culture for 24 hours. And then TGF- β1 (5ng/ml) induction was added, and at the same time different concentration gradients of Carrimycin and Isovalerylspiramycin I were added. The concentrations were 20µmol/L and 40µmol/L. The following groups were set, control group (without adding TGF -β1 induction), TGF-β1 induction group (only TGF-β1 induction is added) and TGF-β1 induction administration group (both adding TGF-β1 induction and treating with Carrimycin or Isovalerylspiramycin I).

After culturing for 24 hours, the culture medium was discarded, and the Total RNA of LX-2 cells were extracted according to the operation steps of the TRIzol instructions. According to the operation steps of the instructions of Roche Transcriptor First Strand cDNA Synthesis Kit, Total RNA of LX-2 was reversely transcribed into cDNA. The cDNA, sterile water, Roche FastStart Universal Probe Master (Rox) and ABI TaqMan probes (GAPDH, COL1A1, TGFB1, ACTA2, MMP2) were prepared into a 20µl reaction system, and the ABI 7500 Fast Real-time PCR System was used for detection. GAPDH was used as an internal control to analyze the results. It is showed by the data, both Carrimycin and Isovalerylspiramycin I can significantly inhibit COL1A1, TGFB1, ACTA2, and MMP2 (Figure 11). Isovalerylspiramycin I was obviously toxic to CCC-ESF-1 cells at a concentration of 40 µmol/L.

The above are only preferred embodiments of the present disclosure, and do not limit the present disclosure in any form. Although the present disclosure has been disclosed as preferred embodiments, it is not intended to limit the present disclosure. Without departing from the technical solution of the present disclosure, any person familiar with this patent can make some changes or modifications to equivalent embodiments with equivalent changes by using the technical contents indicated above, but any simple modifications, equivalent changes and modifications made to the above embodiments according to the technical essence of the present disclosure without departing from the technical solution of the present disclosure, still fall within the scope of the present disclosure.

## Claims

1. A medicament for preventing, alleviating and/or treating fibrosis, comprising an effective component comprising one of Carrimycin, Isovalerylspiramycin I, Isovalerylspiramycin II and Isovalerylspiramycin III;
or a combination of two or three of Isovalerylspiramycin I, Isovalerylspiramycin II and Isovalerylspiramycin III.

2. The medicament for preventing, alleviating and/or treating fibrosis according to claim 1, wherein the medicament comprises a pharmaceutically acceptable carrier.

3. A medicament for preventing, alleviating and/or treating fibrosis, comprising an effective component comprising one or more selected from a group consisting of:
a derivative, a pharmaceutically acceptable salt, a solvate, a metabolite, a stereoisomer, a tautomer, a polymorph and a drug precursor of Carrimycin;
a derivative, a pharmaceutically acceptable salt, a solvate, a metabolite, a stereoisomer, a tautomer, a polymorph and a drug precursor of Isovalerylspiramycin III;
a derivative, a pharmaceutically acceptable salt, a solvate, a metabolite, a stereoisomer, a tautomer, a polymorph and a drug precursor of Isovalerylspiramycin II; and
a derivative, a pharmaceutically acceptable salt, a solvate, a metabolite, stereoisomer, a tautomer, a polymorph and a drug precursor of Isovalerylspiramycin I.

4. A combination product for treating fibrosis, comprising a first drug, wherein an effective component of the first drug comprises one of Carrimycin, Isovalerylspiramycin I, Isovalerylspiramycin II and Isovalerylspiramycin III;
or a combination of two or three of Isovalerylspiramycin I, Isovalerylspiramycin II and Isovalerylspiramycin III.

5. The combination product for treating fibrosis according to claim 4, wherein the combination product further comprises a second drug, and the second drug comprises at least one of medicaments for preventing, alleviating and/or treating fibrosis.

6. The combination product for treating fibrosis according to claim 5, wherein the medicaments for preventing, alleviating and/or treating fibrosis include corticosteroids, colchicine, silymarin, or interferon.

7. A combination product for treating fibrosis, comprising a first drug, an effective component of the first drug comprises one or more selected from a group consisting of:
a derivative, a pharmaceutically acceptable salt, a solvate, a metabolite, a stereoisomer, a tautomer, a polymorph and a drug precursor of Carrimycin;
a derivative, a pharmaceutically acceptable salt, a solvate, a metabolite, a stereoisomer, a tautomer, a polymorph and a drug precursor of Isovalerylspiramycin III;
a derivative, a pharmaceutically acceptable salt, a solvate, a metabolite, a stereoisomer, a tautomer, a polymorph and a drug precursor of Isovalerylspiramycin II; and
a derivative, a pharmaceutically acceptable salt, a solvate, a metabolite, stereoisomer, a tautomer, a polymorph and a drug precursor of Isovalerylspiramycin I.

8. The medicament according to any one of claims 1-3 or the combination product according to any one of claims 4-7, wherein the fibrosis includes pulmonary fibrosis, cardiac fibrosis, liver fibrosis, pancreatic fibrosis, kidney fibrosis, bone marrow fibrosis and skin fibrosis;
preferably, the pulmonary fibrosis includes pulmonary fibrosis caused by a novel coronavirus infection.

9. Use of the medicament according to any one of claims 1-3 or the combination product according to any one of claims 4-7 in preventing, alleviating and/or treating fibrosis.

10. Use of the medicament according to any one of claims 1-3 or the combination product according to any one of claims 4-7 in inhibiting inflammation or lipid peroxidation, inhibiting proliferation and activation of fibroblasts, and promoting collagen degradation.
